# EUROPEAN PATENT APPLICATION

(11) **EP 3 845 513 A1**
(43) Date of publication of application: **07.07.2021**
(21) Application number: 20201205.0
(22) Date of filing: 12.10.2020
(51) Int. Cl.: C07C 2/80, C07C 11/24, B01J 12/00, B01J 19/08

(54) **A METHOD OF PREPARING ACETYLENE (C2H2)**

(71) Applicant: Shell Internationale Research Maatschappij B.V., 2596 HR The Hague (NL)
(72) Inventor: VAN BAVEL, Alexander Petrus, Amsterdam (NL); DE, Shauvik, Bangalore (IN)
(74) Representative: Shell Legal Services IP

(57) **Abstract**

The present invention provides a method of preparing acetylene (C₂H₂), the method at least comprising the steps of:
a) providing a hydrocarbon-containing stream; and
b) subjecting the hydrocarbon-containing stream provided in step a) to pyrolysis at a temperature below 2500°C, thereby obtaining at least acetylene (C₂H₂) and hydrogen (H₂);
wherein, in step b), the hydrocarbon-containing stream is heated by passing it through a structured packing, which structured packing is resistively heated by means of direct electric heating.

## Description

The present invention relates to a method of preparing acetylene (C₂H₂).

Various methods of preparing acetylene are known in the art.

As a mere example, the article by H. Schobert in Chem. Rev. 2014, 114, pages 1743-1760 provides an overview of the production of acetylene and acetylene-based chemicals from coal. A problem associated with coal-based processes for preparing acetylene is that several contaminants appear in the (intermediate) products, as a result of which the products need further purification and the recycling of (intermediate) products is not optimal.

Further, it has been proposed to convert methane into acetylene using electric arcs (a form of plasma technology). Problems associated with the use of electric arcs are, amongst others, scale-up problems and residence time control (resulting in either 'over-conversion' into coke or 'under-conversion').

It is an object of the present invention to overcome or minimize one or more of the above problems.

It is a further object of the present invention to provide an alternative method for producing acetylene, resulting in improved residence time control.

One or more of the above or other objects can be achieved by a method of preparing acetylene (C₂H₂), the method at least comprising the steps of:
a) providing a hydrocarbon-containing stream; and
b) subjecting the hydrocarbon-containing stream provided in step a) to pyrolysis at a temperature below 2500°C, thereby obtaining at least acetylene (C₂H₂) and hydrogen (H₂);
wherein, in step b), the hydrocarbon-containing stream is heated by passing it through a structured packing, which structured packing is resistively heated by means of direct electric heating.

It has surprisingly been found according to the present invention that it allows for an improved residence time and temperature control in the structured packing where the pyrolysis takes place, which may result in an improved conversion and/or selectivity and hence less by-products.

A further advantage of the method according to the present invention is that less energy for the heating is required as the heating takes place in a more targeted manner with a better temperature control (and lower temperature variation). Also, faster temperature adjustment can be achieved, e.g. when the composition of the hydrocarbon-containing stream is changed over time.

Another advantage of the method according to the present invention is that less downtime is expected. Also, the lifetime of the equipment used may be higher as more targeted and less severe conditions are used (such as lower temperature). Also, in view of the relatively simple equipment used, maintenance may be simpler.

In step a) of the method according to the present invention, a hydrocarbon-containing stream is provided. The person skilled in the art will readily understand that this hydrocarbon-containing stream can vary widely and may contain additional components dependent on the origin. Preferably, the hydrocarbon-containing stream is a gaseous stream. Typical examples of the hydrocarbon-containing stream are natural gas, fuel gas, refinery off-gas, etc.

Preferably, the hydrocarbons in the hydrocarbon-containing stream are predominantly C₁-C₅ hydrocarbons (such as methane, ethane, propane, butane and pentane).

Generally, the hydrocarbon-containing stream provided in step a) has a total hydrocarbon content of at least 30 mol.%, preferably at least 40 mol.%, more preferably at least 50 mol.%.

Typically, the hydrocarbon-containing stream provided in step a) comprises at least 30 mol.% methane, preferably at least 50 mol.%, more preferably at least 70 mol.%, even more preferably at least 90 mol.% methane.

Further, it is preferred that the hydrocarbon-containing stream provided in step a) comprises at most 10 mol.% nitrogen (N₂), more preferably at most 5 mol.%, preferably at most 2 mol.%. Also, it is preferred that the hydrocarbon-containing stream comprises at most 5 mol.% CO₂, preferably at most 1 mol.%. Further, it is preferred that the hydrocarbon-containing stream comprises at most 1 mol.% CO.

In step b), the hydrocarbon-containing stream provided in step a) is subjected to pyrolysis at a temperature below 2500°C, thereby obtaining at least acetylene (C₂H₂) and hydrogen (H₂);
wherein, in step b), the hydrocarbon-containing stream is heated by passing it through a structured packing, which structured packing is resistively heated by means of direct electric heating.

As the person skilled in the art is familiar with pyrolysis, this is not discussed here in detail.

The person skilled in the art will readily understand that - dependent on inter alia the nature of the hydrocarbon feed stream, the pyrolysis conditions and the residence time - the product stream comprising at least acetylene (C₂H₂) and hydrogen (H₂) may vary widely. As an example, the product stream may comprise other components such as ethylene. Preferably, the product stream comprises at least 2 mol.% acetylene, preferably at least 10 mol.% acetylene. Generally, the product stream comprises at most 5 mol.% solid carbon, typically at most 2 mol.% and preferably below 1 mol.%.

The person skilled in the art will readily understand that the pyrolysis of step b) can be performed at a wide range of temperatures provided that it is performed at a temperature below 2500°C (to minimize 'over-conversion' into elemental carbon such as coke as may take place when using electric arcs at higher temperatures). Preferably, the pyrolysis is non-catalytic pyrolysis.

Generally, the pyrolysis of step b) is performed at a temperature of at least 800°C. Preferably, the pyrolysis of step b) is performed at a temperature of at least 900°C, preferably at least 1000°C, more preferably at least 1500°C, even more preferably at least 1700°C and preferably at most 2200°C.

The person skilled in the art will readily understand that the pyrolysis of step b) can be performed at a wide range of pressures. Generally, the pyrolysis of step b) is performed at a pressure of from 0.1 to 20 bara. Preferably, the pyrolysis of step b) is performed at a pressure in the range of from 0.9 to 10.0 bara, more preferably from 1.0 to 5.0 bara.

One of the advantages of the method of the present invention is that it allows for a well specified short residence time in the area where the pyrolysis takes place (i.e. in the structured packing). This well specified short residence time results in minimization of coke formation.

Typically, the hydrocarbon-containing stream has a residence time in the structured packing of at most 2 ms and at least 0.1 ms. Preferably, the hydrocarbon-containing stream has a residence time in the structured packing of at most 1 ms, preferably in the range of from 0.2 to 0.6 ms. Further, it is preferred that the variation of the residence time in in the structure packing is low, i.e. less than 20%, preferably less than 10%, more preferably less than 5%. This allows for a targeted pyrolysis under the prevailing pyrolysis conditions.

The person skilled in the art will readily understand that the structured packing as used in the present invention can vary widely. As an example, the structured packing may be composed of plates, sheets, wires, gauzes, meshes, etc. arranged in such a way that it defines a (preferably non-linear) flow path for the hydrocarbon-containing stream whilst creating a large surface area for contact with the structured packing but still allowing the hydrocarbon-containing stream to flow easily. The plates and sheets (when used) may be corrugated and perforated and may contain other surface enhancements.

If desired, the structured packing may be modular, i.e. containing at least two or more separate elements (which may be the same or different) in series. Preferably, the at least two or more separate elements are independently heated. The independently heated separate elements may have the same or a different temperature. An advantage of the modular set-up of the structured packing is that certain elements may be temporarily removed for cleaning and maintenance purposes (such as removing coke formation that has been attached to it during the pyrolysis process) without leading to increased downtime of the reactor. Another advantage of the modular set-up is that it allows even better temperature control. Another advantage of the modular set-up is that it can be scaled up easily.

Typically, the structured packing has a porosity of at least 0.25.

The material of the structured packing is not particularly limited, provided that it can generate the direct electric heating and withstand the intended temperatures and other conditions of the pyrolysis. As mere examples, suitable materials may be metals, alloys or ceramic materials, such as tungsten (W), Fe-Cr alloys, (doped) SiC and doped Al₂O₃. Generally, the structured packing (as a whole) will have a resistivity of at least 1.0 x 10⁻⁸ Ω·m (Ohm·m) at 20°C, preferably at least 4.0 x 10⁻⁷ Ω·m at 20°C, more preferably at least 2.0 x 10⁻⁶ Ω·m at 20°C, and typically below 1.0 x 10⁻⁴ Ω·m at 20°C. The structured packing may be coated with a catalytically active material.

To allow the pyrolysis to occur in step b) the structured packing is resistively heated by means of direct electric heating (sometimes also referred to with 'Joule heating', 'Ohmic heating' or 'impedance heating'). The direct electric heating may be achieved by AC or DC current. The advantage of the direct electric heating is that the temperature can be targeted and controlled within a narrow range.

Preferably, renewable electricity (i.e. from a renewable source such as wind energy, solar energy, etc.) is used for the direct electric heating.

The person skilled in the art will understand that in addition to the direct electric heating some further (supporting) heating may take place as well, e.g. via the walls of the reactor, pre-heating of the hydrocarbon-containing stream, etc.

According to a preferred embodiment according to the present invention, the structured packing has a temperature variation of at most 100°C, preferably at most 50°C, more preferably at most 20°C. As mentioned above, the advantage of the direct electric heating is that the temperature can be targeted and controlled within a narrow range.

In another aspect, the present invention provides a reactor for preparing acetylene (C₂H₂) from a hydrocarbon-containing stream according to the method of the present invention, the reactor at least comprising:
- an inlet for a hydrocarbon-containing stream and an outlet for acetylene (C₂H₂) and hydrogen (H₂);
- a structured packing placed in the reactor, defining a flow path for the hydrocarbon-containing stream; and
- an electric circuit connected to the structured packing, wherein during use the electric circuit can resistively heat the structured packing in the reactor to a temperature of from 800°C to 2500°C by means of direct electric heating.

The person skilled in the art will readily understand that the reactor may vary widely.

Preferably, the reactor is a tubular reactor.

Further, it is preferred that the reactor further comprises a quench zone downstream of the structured packing, the quench zone having an increased diameter compared to the structured packing. If desired, the quench zone may comprise one or more inlets for a quench fluid such as water or (light) hydrocarbons.

Hereinafter the present invention will be further illustrated by the following non-limiting drawing. Herein shows:

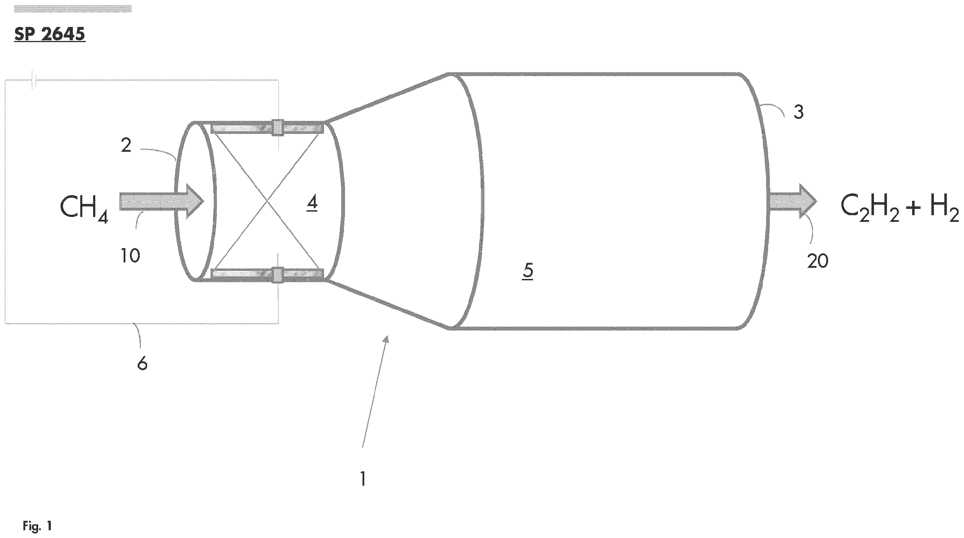

Fig. 1 schematically an embodiment of the reactor according to the present invention.

As shown in the embodiment of Fig. 1, the reactor 1 comprises an inlet 2 (for providing a hydrocarbon-containing stream 10) and an outlet 3 (for product stream 20 containing at least acetylene (C₂H₂) and hydrogen (H₂)), a structured packing 4 placed in the reactor 1, a quench zone 5 and an electric circuit 6 connected to the structured packing 4.

If desired, the structured packing 4 may be modular, i.e. containing at least two or more separate elements (not shown in Fig. 1) in series. These at least two or more separate elements are preferably independently heated.

The structured packing 4 defines a (preferably non-linear) flow path for the hydrocarbon-containing stream 10 whilst creating a large surface area for contact with the structured packing 4 but still allowing the hydrocarbon-containing stream 10 to flow easily through the structured packing 4.

The electric circuit 6 can, during use, resistively heat the structured packing 4 in the reactor 1 by means of direct electric heating.

In the embodiment of Fig. 1, the reactor 1 is a tubular reactor. The quench zone 5 is positioned downstream of the structured packing 4 and has an increased diameter compared to the diameter of the structured packing 4. The quench zone 5 may comprise one or more inlets (not shown) for a quench fluid such as water.

During use of the reactor 1 according to the present invention, the hydrocarbon-containing stream 10 is subjected to pyrolysis in the structured packing 4 at a temperature below 2500°C, thereby obtaining a product stream 20 containing at least acetylene (C₂H₂) and hydrogen (H₂). To this end, the hydrocarbon-containing stream 10 is heated by passing it through the structured packing 4, which structured packing 4 is resistively heated by means of direct electric heating as provided by the electric circuit 6.

The use of the direct electric heating allows for a targeted temperature control in the structured packing 4. As a result, the residence time of the hydrocarbon-containing stream 10 in the structured packing 4 can be kept short and well defined (with only a small variation in residence time and temperature), and is preferably at most 1 ms, preferably in the range of from 0.2 to 0.6 ms.

The person skilled in the art will readily understand that many modifications may be made without departing from the scope of the invention.

## Claims

1. A method of preparing acetylene (C₂H₂), the method at least comprising the steps of:
a) providing a hydrocarbon-containing stream; and
b) subjecting the hydrocarbon-containing stream provided in step a) to pyrolysis at a temperature below 2500°C, thereby obtaining at least acetylene (C₂H₂) and hydrogen (H₂);
wherein, in step b), the hydrocarbon-containing stream is heated by passing it through a structured packing, which structured packing is resistively heated by means of direct electric heating.

2. The method according to claim 1, wherein the hydrocarbon-containing stream provided in step a) comprises at least 30 mol.% methane, preferably at least 50 mol.%, more preferably at least 70 mol.%, even more preferably at least 90 mol.% methane.

3. The method according to claim 1 or 2, wherein the pyrolysis of step b) is performed at a temperature of at least 900°C, preferably at least 1000°C, more preferably at least 1500°C, even more preferably at least 1700°C and preferably at most 2200°C.

4. The method according to any one of the preceding claims, wherein the pyrolysis of step b) is performed at a pressure in the range of from 0.9 to 10.0 bara, preferably from 1.0 to 5.0 bara.

5. The method according to any one of the preceding claims, wherein the hydrocarbon-containing stream has a residence time in the structured packing of at most 1 ms, preferably in the range of from 0.2 to 0.6 ms.

6. The method according to any one of the preceding claims, wherein the structured packing has a temperature variation of at most 100°C, preferably at most 50°C, more preferably at most 20°C.

7. A reactor for preparing acetylene (C₂H₂) from a hydrocarbon-containing stream according to the method of any of the claims 1-6, the reactor at least comprising:
- an inlet for a hydrocarbon-containing stream and an outlet for acetylene (C₂H₂) and hydrogen (H₂);
- a structured packing placed in the reactor, defining a flow path for the hydrocarbon-containing stream; and
- an electric circuit connected to the structured packing, wherein during use the electric circuit can resistively heat the structured packing in the reactor to a temperature of from 800°C to 2500°C by means of direct electric heating.

8. The reactor according to claim 7, wherein the reactor is a tubular reactor.

9. The reactor according to claims 7 or 8, further comprising a quench zone downstream of the structured packing, the quench zone having an increased diameter compared to the structured packing.
